**Europäisches Patentamt**

**European Patent Office**

**Office européen des brevets**

(19)

(11) Publication number: **0 233 853 B1**

(12) **EUROPEAN PATENT SPECIFICATION**

(45) Date of publication of the patent specification:
**12.09.90**

(51) Int. Cl.⁵: **A61K 9/46**

(21) Application number: **87870007.9**

(22) Date of filing: **20.01.87**

(54) Effervescent couples, effervescent compositions of H2-antagonists of histamine and their preparation.

(30) Priority: **22.01.86 FR 8600839**

(43) Date of publication of application:
**26.08.87 Bulletin 87/35**

(45) Publication of the grant of the patent:
**12.09.90 Bulletin 90/37**

(84) Designated Contracting States:
**AT BE CH DE ES FR GB GR IT LI LU NL SE**

(56) References cited:
**FR-A- 1 484 202**
**FR-A- 2 092 893**
**FR-A- 2 132 521**

(73) Proprietor: **Laboratoires SMITH KLINE & FRENCH, 12, Place de la Défense, Cedex 26, F-92090 Paris-la-Défense(FR)**

(72) Inventor: **Tarral, René, 3, Rue Georges Ville, F-75016 Paris(FR)**
Inventor: **Mention, Jacky, 4, rue du Maréchal Joffre, F-33850 Leognan(FR)**

(74) Representative: **Waters, David Martin, Dr. et al, Smith Kline & French Laboratories Ltd. Patent Department Mundells, Welwyn Garden City Hertfordshire AL7 1EY(GB)**

## Description

La présente invention concerne des couples effervescents, des compositions effervescentes d'antagonistes H2 de l'histamine les contenant et leur préparation. Ces compositions se présentent sous forme de poudres et de comprimés qui se dissolvent avec effervescence.

L'histamine, composé physiologiquement actif, endogène chez les mammifères, agit par interaction avec certains sites appelés récepteurs. Un type de récepteur est connu comme récepteur H1 de l'histamine (Ash and Schild, Brit. J. Pharmac. Chemother. 27 427, 1966) et les actions de l'histamine dues à l'action médiatrice de ces récepteurs sont bloquées par les médicaments appelés couramment "antihistamines" (antagonistes H1 de l'histamine) dont un exemple courant est la mépyramine. Un second type de récepteur d'histamine est connu comme récepteur H2 (Black et al., Nature 236, 385, 1972). Ces récepteurs ne sont pas bloqués par la mépyramine mais ils sont bloqués par la burimamide. Les composés qui bloquent les récepteurs H2 de l'histamine sont appelés antagonistes H2 de l'histamine.

Comme antagonistes H2 de l'histamine classiques, on peut citer la cimétidine, la famotidine, la ranitidine, la nizatidine, l'étintidine, la lupitidine, la mifentidine, la niperotidine, la roxatidine, la sufotidine, la tuvatidine et la zaltidine.

Ces produits sont utiles dans le traitement des ulcérations de la muqueuse gastro-duodénale (ulcère duodénal, ulcère gastrique bénin, ulcère récidivant et ulcère peptique post-chirurgicaux), de l'oesophagite peptique, des hémorragies résultant d'ulcérations ou d'érosions oesophagiennes, gastriques ou duodénales et du syndrome de Zollinger-Ellison.

Chez les patients ayant des antécédents d'ulcération duodénale récurrente, ils sont utiles pour la prévention des récidives.

Pour l'administration orale, les antagonistes H2 de l'histamine peuvent se présenter sous forme de comprimés ou sous forme de suspension aqueuse.

Ainsi, la demande de brevet européen publiée sous la No. 0 138 540 décrit des compositions pour administration orale de cimétidine qui se présentent sous forme de suspensions aqueuse éventuellement aromatisées pour atténuer l'amertume du produit; ces compositions peuvent également contenir un antacide, c'est-à-dire un produit pharmaceutiquement acceptable de nature alcaline et pouvant, comme le carbonate de calcium par exemple, neutraliser l'acidité gastrique.

Contrairement aux formes précédemment connues pour l'administration d'antagonistes H2 de l'histamine par voie orale et notamment celles de la demande de brevet européen No. 0 138 540, les compositions de la présente invention ne sont pas administrées telles quelles mais, extemporanément, elles sont versées dans un véhicule aqueux (de l'eau de boisson, par exemple) où elles sont effervescentes et entraînent une dissolution rapide du produit antagoniste H2 de l'histamine. Sous une forme préférée, les compositions pharmaceutiques suivant l'invention forment des solutions limpides qui peuvent être aisément aromatisées et offrent une forme d'administration particulièrement agréable.

Les compositions de l'invention se caractérisent par une stabilité particulièrement bonne pour l'antagoniste H2 (par exemple, la cimétidine). Etant donné qu'elles ne sont pas liées à des exigences de volume comme c'est plus particulièrement le cas pour les comprimés à croquer ou à avaler , les préparations pharmaceutiques de l'invention peuvent également contenir des quantités importantes d'antacide, comme par exemple de l'hydroxyde d'aluminium ou de magnésium ou un carbonate ou bicarbonate alcalin ou alcalino-terreux, de préférence, un bicarbonate alcalin. Il a en effet été constaté que, par rapport à la présentation comprimé classique, les compositions effervescentes de l'invention contenant un bicarbonate alcalin n'affectent pas la biodisponibilité de l'antagoniste H2 mais entraînent l'accélération de son absorption.

Dans le but de comparer les biodisponibilités relatives de comprimés classiques et de comprimés effervescents préparés suivant la présente invention, on a utilisé des comprimés dosés à 800 mg de cimétidine sous forme de chlorhydrate préparés suivant la présente invention. Les comprimés ont été administrés en deux passages distincts à 12 sujets à raison d'une dose orale toutes les 24 heures pendant 4 jours consécutifs. Les sujets étaient des 2 sexes, leur âge moyen était de 25 ans (± 1 an) et leur poids de 62 kg (± 3 kg) et, avant leur acceptation pour l'étude, ils avaient subi des prélèvements dans le but de réaliser des contrôles sanguins dont les résultats n'ont permis de déceler aucune anomalie.

A 10 jours d'intervalle, les sujets ont reçu le traitement à base de comprimés traditionnels et le traitement aux comprimés effervescents . L'administration a été faite dans chaque cas à 19 heures au milieu du repas du soir, ce dernier se composant d'une soupe de légumes, de 2 oeufs durs mayonnaise, de salade verte et d'une salade de fruits.

La cimétidine a été dosée par chromatographie liquide à haute performance dans le plasma et les urines des sujets.

Dans le plasma, les analyses ont montré que, alors que les valeurs moyennes de Cmax (caractéristiques du pic des concentrations plasmatiques observé après les première et quatrième administrations des 2 formes de comprimés) sont voisines (elles ne diffèrent respectivement que de 3 % et 8 % au jour 1 et au jour 4), les valeurs moyennes des concentrations plasmatiques de cimétidine (ug/ml$^{-1}$), mesurées durant la première heure suivant la première et la quatrième administration du comprimé effervescent et indiquées dans le Tableau suivant, sont nettement supérieures à celles observées après la prise du comprimé classique.

| Temps | Comprimé classique | | Comprimé effervescent | |
|---|---|---|---|---|
| (h) | 1er jour | 4ème jour | 1er jour | 4ème jour |
| 0,25 | 0,053 | 0,135 | 1,791 | 1,600 |
| | (0,026) | (0,091) | (0,242) | (0,382) |
| 0,50 | 0,492 | 0,312 | 3,176 | 2,506 |
| | (0,146) | (0,148) | (0,376) | (0,345) |
| 0,75 | 1,532 | 0,937 | 3,088 | 2,459 |
| | (0,515) | (0,332) | (0,322) | (0,224) |
| 1,00 | 2,071 | 1,372 | 3,042 | 2,312 |
| | (0,362) | (0,427) | (0,248) | (0,183) |

N.B. : Les valeurs indiquées entre parenthèses sont les valeurs de l'écart type

Les résultats indiquent clairement que la cimétidine est absorbée plus rapidement à partir des comprimés effervescents que à partir des comprimés classiques.

Dans l'urine, la quantité totale de cimétidine excrétée entre 0 et 24 heures et entre 72 et 96 heures s'est révélée pratiquement la même pour les deux types de comprimés.

Selon que la composition se présente sous forme de poudre ou de comprimé, elle pourra également contenir différents excipients comme il est bien connu dans la technique; de tels produits sont les liants et les lubrifiants (par exemple, polyvinylpyrrolidone, benzoate de sodium, polyéthylène glycol, L-leucine ou sulfate de laurylmagnésium) et les agents d'aromatisation, d'édulcoration et de coloration; parmi eux, le benzoate de sodium siliconé - considéré ici comme lubrifiant - s'est révélé particulièrement intéressant.

Les principes de la préparation de compositions pharmaceutiques effervescentes sont connus (voir par exemple Pharmaceutical Dosage Forms (Tablets / Vol. 1) par Lieberman H.A. et Lachman L., éditions M. Dekker, New York, 1980, chapitre 5, Effervescent Tablets par R. Mohrle, p. 255-258) et différentes techniques ont été décrites pour améliorer la stabilité des compositions ou le moyen de les préparer. Ainsi,

- le brevet français No. 1 484 202 décrit un procédé de préparation d'un composé acide pour compositions effervescentes caractérisé en ce que on fait réagir un polyacide organique à l'état pulvérulent avec du bicarbonate de sodium en une quantité inférieure à la quantité stoechiométrique nécessaire pour la salification complète du polyacide organique (entre 30 et 80%), alors que la présente invention nécessite que le carbonate ou le bicarbonate alcalin soit présent au moins en quantité stoechiométrique.

- le brevet 2.984.543 des Etats-Unis d'Amérique décrit un procédé pour stabiliser des compositions bicarbonatées effervescentes sous forme de poudre dans lequel le produit générateur d'anhydride carbonique (c'est-à-dire un carbonate ou bicarbonate sous une forme finement divisée) est imprégné d'un mucilage ou d'une solution d'une gomme hydrophile naturelle ou synthétique.

- le brevet 2.985.562 des Etats-Unis d'Amérique décrit un procédé d'amélioration des caractéristiques de fluidité de compositions effervescentes par ajout d'une petite quantité d'un acide aminé monocarboxy-lique suivi d'un traitement thermique entre 60 et 120° C.

- le brevet 3.105.792 des Etats-Unis d'Amérique décrit un procédé d'amélioration des caractéristiques physiques de comprimés effervescents par un traitement thermique préliminaire du bicarbonate, traitement au cours duquel la surface des particules de bicarbonate est transformée en carbonate correspondant.

- le brevet 3.875.073 des Etats-Unis d'Amérique revendique l'addition d'une protéine pour stabiliser les mélanges effervescents contre l'humidité.

- les brevets 3.773.922 et 3.946.996 des Etats-Unis d'Amérique, la demande de brevet PCT 84/02468 et le brevet belge 781.358 décrivent divers appareillages ou procédés de préparation de comprimés ou granulés effervescents.

- le brevet belge No. 760.288 décrit des poudres et comprimés effervescents préparés à partir de carbonate ou de bicarbonate et d'acide où le composant acide est constitué par du citrate di-acide monosodique pur.

- le brevet européen No. 0 011 489 décrit un procédé de préparation d'une poudre effervescente contenant un analgésique caractérisé en ce que le composant qui provoque l'effervescence est soit du citrate monosodique soit du citrate disodique.

- le brevet européen 0 076 340 revendique une méthode de préparation de granulés effervescents permettant de réaliser une passivation superficielle des granulés par un procédé comprenant plusieurs étapes répétitives.

- la demande de brevet français publiée sous le No. 2 552 308 revendique un mélange effervescent à base d'acide organique solide cristallisé et une source d'anhydride carbonique et dans lequel les cristaux d'acide portent un revêtement protecteur contenant du carbonate de calcium.

- les demandes de brevets britanniques 2 091 625, 2 093 052 et 2 093 376 décrivent différentes variantes d'un appareil permettant la préparation de granulés effervescents et le traitement de leur surface pour augmenter leur résistance à l'humidité.

Il a maintenant été constaté que la préparation de compositions effervescentes au départ d'un mélange d'un antagoniste H2 de l'histamine (la cimétidine), d'une source d'anhydride carbonique (bicarbonate de sodium) et d'un acide (acide citrique) présentait un problème particulier du fait de l'instabilité de l'antagoniste H2 de l'histamine en présence d'acide et que le remplacement de l'acide citrique par du citrate monosodique n'améliorait pas d'une matière satisfaisante la stabilité de l'antagoniste H2 de l'histamine alors que le remplacement de l'acide citrique par du citrate disodique diminuait fortement l'effervescence de la solution et le temps de solubilisation de l'antagoniste H2 de l'histamine.

Néanmoins, et ceci constitue l'objet de la présente invention, il s'est avéré possible d'obtenir le maintien d'une effervescence acceptable sans nuire à la stabilité de l'antagoniste H2 de l'histamine à condition que l'acide citrique soit présent sous la forme d'un couple citrate mono-sodique ou -potassique/citrate di-sodique ou -potassique dans un rapport pondéral compris entre 8/1 et 1/10 et de préférence compris entre 2/1 et 6/10.

Par exemple, lorsqu'on prépare un comprimé effervescent de cimétidine contenant de l'acide citrique, on constate une dégradation immédiate de la cimétidine et lorsqu'on utilise du citrate monosodique pur à la place d'acide citrique, on constate un dégradation de 38 % après une semaine à 70° C alors que, dans les mêmes conditions, on ne constate aucune dégradation de la cimétidine avec un couple de la présente invention.

La notion de 'couple' est définie ici comme le produit obtenu par granulation suivie de séchage d'un mélange d'un acide polycarboxylique et d'une source d'anhydride carbonique de nature alcaline en quantité au moins stoechiométrique par rapport à l'acidité du milieu mais dans des conditions opératoires telles que l'acide ne soit que partiellement neutralisé.

Les couples suivant l'invention sont des couples antacides effervescents préparés à partir d'un acide polycarboxylique pharmaceutiquement acceptable et d'une source d'anhydride carbonique et caractérisés en ce qu'ils comprennent du citrate mono-sodique ou potassique et du citrate di-sodique ou potassique dans un rapport pondéral compris entre 8/1 et 1/10, de préférence entre 2/1 et 6/10.

Dans le cas de couples préparés au départ de quantités stoechiométriques d'acide citrique et de bicarbonate de sodium, ce résultat est atteint en stoppant la réaction lorsque de 24 à 54 % de la quantité potentielle d'anhydride carbonique a été libérée.

Il est évident que les couples peuvent également contenir différents additifs au même titre que les compositions finales, comme il est indiqué ci-avant. Parmi ces additifs, le benzoate, sous forme de benzoate alcalin micronisé et siliconé s'est révélé particulièrement intéressant.

On remarquera en passant que, dans l'art antérieur, les spécialistes se sont surtout préoccupés de la stabilité du couple acide/source d'anhydride carbonique lui-même en présence d'humidité alors que, dans la présente invention, le premier objectif a été de stabiliser également l'ingrédient médicalement actif.

L'invention est illustrée par les exemples suivants qui ne sont pas limitatifs de l'invention.

EXEMPLE 1

Dans un mélangeur granulateur sécheur comprenant une cuve à double enveloppe thermostatable raccordée à une pompe à vide, un orifice de chargement de matières solides, un orifice pour l'introduction de liquides sous forme dispersée, un outil de mélange pouvant assurer une très bonne homogénéité et un dispositif de mise à pression atmosphérique et porté à 100° C (± 5° C), on introduit à travers une grille de un millimètre d'ouverture de maille, 43,250 kg d'acide citrique anhydre en poudre et 56,750 kg de bicarbonate de sodium. Sous agitation énergique le mélange est chauffé. Lorsque la température des produits atteint 40° C (± 1° C), le thermostat de la double enveloppe est réglé sur 75° C (± 1° C).

Lorsque la température du mélange atteint 45° C (± 1° C), on introduit sous pression réduite (600 mm Hg) 160 ml d'eau et on ouvre le dispositif de mise à pression atmosphérique. On laisse réagir pendant 40 minutes en maintenant une agitation énergique. Ensuite, on diminue l'agitation et on bloque la réaction par séchage sous aspiration énergique et continue, ce qui se traduit par un abaissement de la pression résiduelle et de la température (de l'ordre de 5 à 10° C), celle-ci remontant ensuite dès que la masse est sèche.

On casse alors le vide et on calibre la masse sur une grille de deux millimètres d'ouverture de maille.

On récupère 78,5 kg de couple stoechiométrique présentant une activité effervescente (exprimée en pourcentage du potentiel initial en anhydride carbonique) de 64,2 %, correspondant à une composition de 32,2 % en citrate monosodique, 31,4 % en citrate disodique et 36,4 % en bicarbonate de sodium, tous les pourcentages étant exprimés en poids/poids et le dosage de l'anhydride carbonique étant réalisé par volumétrie sur base d'un étalonnage établi à l'aide de quantités connues d'acide citrique et de bicarbonate de sodium.

EXEMPLE 2

Dans une atmosphère contrôlée en température et humidité relative (20° C (± 1° C) et 20 % (± 1 %) d'humidité relative), on mélange intimement un kg de cimétidine, 15 kg du couple obtenu à la fin de l'exemple 1 et 700 g de benzoate de sodium micronisé.

On comprime le mélange sous presse alternative équipée de poinçons ronds plats de 20 mm de diamètre au poids de 1,670 g pour obtenir des comprimés dosés à 100 mg de cimétidine.

Les comprimés sont conditionnés en sachets formés par scellage de feuilles de complexe Kraft/polyéthylène (12,5 g)/aluminium (30 um)/polyéthylène (25 g).

Pour évaluer la stabilité des comprimés, des sachets ont été stockés pendant 7 jours en étuve à 70° C (± 1° C) et pendant 10 jours en étuve à 60° C (± 1° C) et, par chromatographie en phase liquide à haute performance (HPLC) sur colonne PARTISIL®-10 SCX (une résine échangeuse de cations fabriquée et vendue par WHATMAN, Inc., Clifton, N.J., U.S.A.) en utilisant comme phase mobile le mélange sulfate d'ammonium 0,075 M (65 volumes); méthanol pour spectroscopie (35 volumes); ammoniaque qsp pH 7, on a recherché la présence éventuelle de produits de dégradation de la cimétidine, à savoir la N-carbamyl-N'-méthyl-N'-[2-(5-méthyl-4-imidazolylméthylthio)éthyl]-guanidine et la N-méthyl-N'-[2-(5-méthyl-4-imidazolylméthylthio)éthyl]-guanidine. Dans ces conditions, aucune trace de produit de dégradation de la cimétidine n'a pu être mise en évidence, démontrant ainsi la parfaite stabilité des comprimés.

Par contre, la préparation de comprimés au départ d'un simple mélange de cimétidine (un kg), de benzoate de sodium (0,700 kg) et d'un mélange présentant une activité effervescente identique (63,2 %) à celle du couple obtenu à la fin de l'exemple 1 et constitué de citrate monosodique (4,575 kg), de citrate disodique (5,050 kg) et de bicarbonate de sodium (5,385 kg) a présenté dans les mêmes conditions de compression et de stockage une teneur de 5,8 % en N-carbamyl-N'-méthyl-N'[2-(5-méthyl-4-imidazolylméthylthio)éthyl]-guanidine et 0,5 % en N-méthyl-N'-[2-(5-méthyl-4-imidazolylméthylthio)éthyl]-guanidine après 7 jours à 70° C et de 8,5 % en N-méthyl-N'-2-(5-méthyl-4-imidazolylméthylthio)éthyl guanidine et 0,6 % en N-méthyl-N'-[2-(5-méthyl-4-imidazolylméthylthioéthyl]-guanidine après 10 jours à 60° C.

EXEMPLE 3

Dans un mélangeur granulateur sécheur comprenant une cuve à double enveloppe thermostatable raccordée à une pompe à vide, un orifice de chargement de matières solides, un orifice pour l'introduction de liquides sous forme dispersée, un outil de mélange pouvant assurer une très bonne homogénéité et un dispositif de mise à pression atmosphérique et porté à 100° C (± 5° C), on introduit à travers une grille de un millimètre d'ouverture de maille, 33,560 kg d'acide citrique anhydre poudre et 44,040 kg de bicarbonate de sodium. Sous agitation énergique, le mélange est chauffé. Lorsque la température des produits atteint 43° C (± 1° C), le thermostat de la double enveloppe est réglé sur 65° C (± 1° C).

Lorsque la température du mélange atteint 51° C (± 1° C), on introduit sous pression réduite (600 mm Hg) 165 ml d'eau et on ouvre le dispositif de mise à pression atmosphérique. On laisse réagir pendant 35 minutes sous agitation énergique. Ensuite, on diminue l'agitation et on bloque la réaction par séchage sous aspiration énergique et continue.

Lorsque la masse est sèche, on casse le vide et on calibre la masse par passage sur une grille de deux millimètres d'ouverture de maille.

On récupère 61,2 kg de couple stoechiométrique présentant une activité effervescente (exprimée en pourcentage du potentiel initial en anhydride carbonique) de 67,8 %, correspondant à une composition de 38,6 % en citrate monosodique, 22,9 % en citrate disodique et 38,5 % en bicarbonate de sodium, tous les pourcentages étant exprimés en poids/poids et le dosage de l'anhydride carbonique étant réalisé par volumétrie sur base d'un étalonnage établi à l'aide de quantités connues d'acide citrique et de bicarbonate de sodium.

EXEMPLE 4

En procédant suivant les conditions opératoires décrites dans l'exemple 2, on a préparé des comprimés de cimétidine avec le couple préparé dans l'exemple 3 et on a étudié leur stabilité en sachets.

Après 7 jours de stockage à 70° C (± 1° C), aucune trace de produits de dégradation (respectivement N-carbamyl-N'-methyl-N'-[2-(5-méthyl-4-imidazolylméthylthio)éthyl]-guanidine et N-méthyl-N'-[2-(5-méthyl-4-imidazolylméthylthio)éthyl]-guanidine) n'a été décelée par chromatographie liquide à haute performance (HPLC).

EXEMPLE 5

Dans un mélangeur granulateur sécheur comprenant une cuve de 130 l de capacité totale (capacité utile : 90 l) équipée d'une double enveloppe pour chauffage par circulation d'eau chaude, raccordée à une pompe à vide et comprenant un orifice de chargement de matières solides, un orifice pour l'introduction de liquides sous forme dispersée, un outil de mélange pouvant assurer une très bonne homogénéité, un couteau rotatif tournant à grande vitesse et un dispositif de mise à pression atmosphérique et porté à 65° C, on introduit sous agitation énergique à travers une grille de un millimètre d'ouverture de maille, 20,975 kg d'acide citrique anhydre en poudre, 27,525 kg de bicarbonate de sodium et 1,500 kg de polyéthylène glycol de poids moléculaire 6.000. Les produits sont démottés à l'aide du couteau rotatif puis chauffés sous agitation énergique. Lorsque la température du mélange atteint 51° C, on ajoute en six fractions égales, à 3 minutes d'intervalle, 300 ml d'eau et on laisse réagir sous agitation énergique 31 minutes à partir de la première addition d'eau.

On bloque alors la réaction par séchage sous aspiration continue, l'homogénéité de la masse étant maintenue par utilisation discontinue de l'outil de mélange à faible vitesse.

Après séchage, on récolte 39,200 kg de couple stoechiométrique présentant une activité effervescente (exprimée en pourcentage du potentiel initial en anhydride carbonique) de 70,3 %, correspondant à une composition de 42,25 % en citrate monosodique, 15,5 % en citrate disodique, 38,65 % en bicarbonate de sodium et 3,6 % en polyéthylène glycol, tous les pourcentages étant exprimés en poids/poids et le dosage de l'anhydride carbonique étant réalisé par volumétrie sur base d'un étalonnage établi à l'aide de quantités connues d'acide citrique et de bicarbonate de sodium.

### EXEMPLE 6

En procédant suivant les conditions opératoires de l'exemple 2, on a préparé des comprimés de cimétidine avec le couple préparé dans l'exemple 5 et on a étudié leur stabilité en sachets.

Après 20 jours de stockage à 40° C (± 1° C), aucune trace de produits de dégradation (respectivement N-carbamyl-N'-méthyl-N'-[2-(5-méthyl-4-imidazolylméthylthio)éthyl]-guanidine et N-méthyl-N'-[2-(5-méthyl-4-imidazolylméthylthio)éthyl]-guanidine n'a été décelée par chromatographie liquide à haute performance (HPLC).

### EXEMPLE 7

Pour fixer les limites d'acceptabilité des préparations effervescentes à base de cimétidine, on a préparé un couple présentant une activité effervescente plus importante que celle des exemples précédents. A cette fin, on a suivi le mode opératoire de l'exemple 1 en ramenant le temps de réaction de 40 minutes à 25 minutes.

On a ainsi obtenu 85 kg d'un couple stoechiométrique présentant une activité effervescente de 78 % et ce couple a été utilisé pour préparer des comprimés de cimétidine, en suivant la technique de l'exemple 2.

Le test de stabilité a mis en évidence une importante dégradation de la cimétidine en N-carbamyl-N'-méthyl-N'-[2-(5-méthyl-4-imidazolylméthylthio)éthyl]-guanidine (0,89 %) dès la fabrication des comprimés et 6 % après une semaine de stockage à 70° C (± 1° C).

D'autre part, un essai de stabilité de comprimés préparés suivant la technique de l'exemple 2 à partir d'un mélange constitué de cimétidine (1 kg), de benzoate de sodium (0,700 kg) et d'un mélange de citrate monosodique (8,405 kg) et de bicarbonate de sodium (6,595 kg) présentant la même activité effervescente que le couple préparé ci-dessus (78 %) a montré, après 7 jours à 70° C, une teneur en produits de dégradation s'élevant à 38 % en N-carbamyl-N'-méthyl-N'-[2-(5-méthyl-4-imidazolylméthylthio)éthyl]-guanidine et 4 % en N-méthyl-N'-[2-(5-méthyl-4-imidazolylméthylthio)-éthyl]-guanidine et, après 10 jours à 50° C, une teneur de 4 % en N-carbamyl-N'-méthyl-N'-[2-(5-méthyl-4-imidazolylméthyl-thio)éthyl]-guanidine.

### EXEMPLE 8

En répétant l'exemple 4 mais après avoir remplacé le benzoate de sodium de l'exemple 2 par la même quantité de benzoate de sodium siliconé à 10 % d'huile de silicone (50 centistokes), on a obtenu des comprimés ne présentant pas de dégradation après 7 jours à 70° C ni après 10 jours à 60° C.

### EXEMPLE 9

L'effet protecteur limité du siliconage du benzoate de sodium a été étudié sur des comprimés obtenus avec le mélange citrate monosodique/bicarbonate de sodium de l'exemple 7.

A cette fin, en suivant la technique de l'exemple 2 mais en remplaçant le couple et le benzoate de sodium qui y sont indiqués par les mêmes quantités respectives de mélange citrate monosodique/bicarbonate de sodium et de benzoate de sodium siliconé à 10 % d'huile de silicone (50 centistokes), on a préparé des comprimés de cimétidine base dont la stabilité a été testée après stockage pendant 7 jours à 70° C, ce qui a indiqué que la teneur en N-carbamyl-N'-méthyl-N'-[2-(5-méthyl-4-imidazolylmé-thylthio)éthyl]-guanidine était ainsi ramenée de 38 % à 12 % et la teneur en N-méthyl-N'-[2-(5-méthyl-4-imidazolylméthylthio)éthyl]-guanidine était ramenée de 4 % à 1,2 %.

De même, l'effet protecteur limité du benzoate de sodium siliconé a été étudié sur des comprimés obtenus suivant la technique de l'exemple 2 avec le mélange citrate monosodique/citrate disodi- que/bicarbonate de sodium indiqué à l'exemple 2. On a ainsi constaté que, après 10 jours de stockage à 60° C, les taux en N-carbamyl-N'-méthyl-N'-[2-(5-méthyl-4-imidazolylméthylthio)éthyl]-guanidine et en N-méthyl-N'[2-(5-méthyl-4-imidazolylméthylthio)éthyl]-guanidine étaient ramenés respectivement de 8,5 % à 6,3 % et de 0,6 % à 0,2 %.

EXEMPLE 10

Dans un mélangeur granulateur sécheur à double enveloppe thermostatable comprenant une cuve raccordée à une pompe à vide, un orifice de chargement de matières solides, un orifice pour l'introduction de liquides, un outil de mélange pouvant assurer une très bonne homogénéité et un dispositif de mise à pression atmosphérique et porté à 100° C, on introduit à travers une grille de un millimètre d'ouverture de maille 33,560 kg d'acide citrique anhydre en poudre et 44,040 kg de bicarbonate de sodium et on chauffe le mélange sous agitation énergique. Lorsque la température des produits atteint 43° C (+ 1° C), le thermostat de la double enveloppe est réglé sur 65° C (± 1° C).

Lorsque la température du mélange atteint 51° C (± 1° C), on procède à l'addition d'eau sous forme pulvérisée par deux ajouts de 155 ml à un intervalle de 5 minutes et, tout en maintenant une vigoureuse agitation, on laisse réagir pendant 50 minutes (mesurées à partir du premier ajout).

On diminue alors la vitesse d'agitation et on bloque la réaction par séchage sous aspiration énergique, ce qui se traduit par un abaissement de la pression résiduelle et de la température (de l'ordre de 5 à 10° C), celle-ci remontant ensuite, lorsque la masse est sèche.

A ce moment, on casse le vide et on calibre la masse par passage sur une grille de deux millimètres d'ouverture de maille.

On récupère ainsi 57,7 kg d'un couple stoechiométrique présentant une activité effervescente de 59 % et correspondant à une composition de 23,1 % en citrate monosodique, 43,4 % en citrate disodique et 33,5 % en bicarbonate de sodium.

EXEMPLE 11

Dans des conditions normales d'ambiance (26° C et 50-55 % d'humidité relative), on mélange intimement 2,500 kg de cimétidine et 25 kg du couple obtenu à la fin de l'exemple 10 et on répartit le mélange dans des sachets formés par scellage de feuilles de complexe Kraft 50 g, polyéthylène 12,5 g, aluminium 30 um et polyéthylène 25 g.

Pour évaluer la stabilité de la poudre ainsi conditionnée, les sachets ont été stockés pendant 6 semaines en étuve à 60° C (± 1° C) et, après cette période, aucun produit de dégradation n'a été décelé par chromatographie en phase liquide à haute performance alors que dans les mêmes conditions de conservation, un mélange ensaché de cimétidine (2,5 kg), acide citrique anhydre en poudre (10,815 kg), bicarbonate de sodium (14,185 kg) a fait apparaître un taux de 20 % en N-carbamyl-N'-méthyl-N'-[2-(5-méthyl-4-imidazolylméthylthio)éthyl]-guanidine et 0,5 % en N-méthyl-N'-[2-(5-méthyl-4-imidazolylméthyl)-thio)éthyl]-guanidine.

Dans les mêmes conditions de conservation, un mélange de cimétidine (2,5 kg), citrate monosodique (12,058 kg) et bicarbonate de sodium (9,456 kg) a montré un taux de 2,3 % en N-carbamyl-N'-méthyl-N'-[2-(5-méthyl-4- imidazolylméthylthio)éthyl]-guanidine et 0,2 % en N-méthyl-N'-[2 (5-méthyl-4-imidazolyl-méthylthio)éthyl]-guanidine.

EXEMPLE 12

Dans un mélangeur granulateur sécheur à double enveloppe thermostatable, comprenant une cuve raccordée à une pompe à vide, un orifice de chargement de matières solides, un orifice pour l'introduction des liquides, un outil de mélange pouvant assurer une très bonne homogénéité et un dispositif de mise à pression atmosphérique et porté à 100° C, on introduit à travers une grille de un millimètre d'ouverture de maille 36,750 kg d'acide citrique poudre anhyde et 63,250 kg de bicarbonate de sodium et on chauffe le mélange sous agitation énergique. Lorsque la température des produits atteint 40° C, le thermostat de la double enveloppe est réglé sur 75° C.

Lorsque la température du mélange atteint 45-46° C, on procède à l'addition de 160 ml d'eau sous forme pulvérisée tout en maintenant une vigoureuse agitation. On laisse réagir pendant 30 minutes (± 3 minutes).

On diminue alors la vitesse d'agitation et on bloque la réaction par séchage sous aspiration énergique ce qui se traduit par un abaissement de la pression résiduelle et de la température (de l'ordre de 5 à 10° C), celle-ci remontant ensuite lorsque la masse est sèche.

A ce moment, on casse le vide et on calibre la masse par passage sur une grille de deux millimètres d'ouverture de maille.

On récupère ainsi 78,4 kg d'un couple avec excès de bicarbonate présentant une activité effervescente de 60,5 % et correspondant à une composition de 24,45 % en citrate monosodique, 28,05 % en citrate disodique, 47,5 % en bicarbonate de sodium.

EXEMPLE 13

En procédant suivant les conditions opératoires de l'exemple 2, on a préparé des comprimés de cimétidine avec le couple proposé dans l'exemple 12 et on a étudié la stabilité en sachets.

Après 13 jours de stockage à 60° C (± 1° C), 0,3 % de N-carbamyl-N'-méthyl-N'-[2-(5-méthyl-4-imidazolylméthylthio)éthyl]-guanidine ont été décelés par chromatographie liquide à haute performance (HPLC).

Par contre, dans les mêmes conditions de conservation des comprimés préparés par simple mélange de cimétidine (1 kg), benzoate de sodium (0,700 kg) avec un mélange présentant une activité effervescente identique à celle du couple précédent et se composant de citrate monosodique (3,740 kg), citrate disodique (4,12 kg), bicarbonate de sodium (7,140 kg) ont montré un taux de dégradation de 10,1 % en N-carbamyl-N'-méthyl-N'-[2-(5-méthyl-4- imidazolylméthylthio)éthyl]-guanidine et de 1,38 % en N-méthyl-N'-[2-(5-méthyl-4-imidazolylméthylthio)éthyl]-guanidine.

EXEMPLE 14

Dans un mélangeur granulateur sécheur à double enveloppe thermostatable, comprenant une cuve raccordée à une pompe à vide, un orifice de chargement de matières solides, un orifice pour l'introduction des liquides, un outil de mélange pouvant assurer une très bonne homogénéité et un dispositif de mise à pression atmosphérique et porté à 100° C, on introduit à travers une grille de un millimètre d'ouverture de maille, 43,250 kg d'acide citrique anhydre poudre et 56,750 kg de bicarbonate de sodium et on chauffe le mélange sous agitation énergique. Lorsque la température des produits atteint 40° C, on règle le thermostat de la double enveloppe à 75° C. Lorsque la température du mélange atteint 45 - 46° C, et que la température de la jaquette est à 75° C, on procède à l'addition de 160 ml d'eau sous forme pulvérisée tout en maintenant une vigoureuse agitation. On laisse réagir pendant 40 minutes. On diminue alors la vitesse d'agitation et on bloque la réaction par un séchage sous aspiration énergique ce qui se traduit par un abaissement de la pression résiduelle et de la température (de l'ordre de 5 à 10° C) celle-ci remontant lorsque la masse est sèche. A ce moment, on casse le vide et on calibre la masse par passage sur une grille de deux millimètres d'ouverture de maille.

On récupère ainsi 78,600 kg d'un couple stoechiométrique présentant une activité effervescente de 62 % et correspondant à une composition de 28,8 % de citrate monosodique, 35,8 % de citrate disodique et 35,4 % de bicarbonate de sodium.

EXEMPLE 15

Dans des conditions d'ambiance contrôlée en température (20° C) et contrôlée en humidité relative (20 %), on mélange intimement 1,1445 partie de chlorhydrate de cimétidine avec 15 parties du couple obtenu à la fin de l'exemple 14 et on répartit le mélange dans des sachets formés par scellage de feuilles de complexe Kraft 50 g, polyéthylène 12,5 g, aluminium 30 um et polyétylène 25 g.

Pour évaluer la stabilité de la poudre ainsi conditionnée, les sachets ont été stockés pendant 1 semaine à 60° C et après cette période, aucun produit de dégradation n'a été décelé par chromatographie en phase liquide à haute performance.

Dans les mêmes conditions de conservation un mélange de
- 1,1445 partie de chlorhydrate de cimétidine
- 8,5125 parties de bicarbonate de sodium
- 6,4875 parties d'acide citrique anhydre poudre
a montré après 1 semaine à 60° C un taux de 59,6 % de N-carbamyl-N'-méthyl-N'-[2-(5-méthyl-4-imidazolylméthylthio)éthyl]-guanidine et 3,5 % de N-méthyl-N'-[2-(5-méthyl-4-imidazolylméthylthio)éthyl]-guanidine.

Dans les mêmes conditions de conservation un mélange de
- 1,1445 partie de chlorhydrate de cimétidine
- 8,410 parties de citrate monosodique
- 6,590 parties de bicarbonate de sodium
a montré après 1 semaine à 60° C un taux de 3,6 % de N-carbamyl-N'-méthyl-N'-[2-(5-méthyl-4-imidazolylméthylthio)éthyl]-guanidine.

Dans les mêmes conditions de conservation un mélange de
- 1,1445 partie de chlorhydrate de cimétidine
- 4,320 parties de citrate monosodique
- 5,380 parties de citrate disodique
- 5,300 parties de bicarbonate de sodium

présentant la même activité (62%) que le couple obtenu à la fin de l'exemple 14 a montré après 1 semaine à 60° C un taux de 3,2 % de N-carbamyl-N'-méthyl-N'-[2-(5-méthyl4-imidazolylméthylthio)éthyl]-guanidine.

## EXEMPLE 16

Dans un mélangeur granulateur sécheur à double enveloppe thermostatable, comprenant une cuve raccordée à une pompe à vide, un orifice de chargement de matière solides, un orifice pour l'introduction des liquides, un outil de mélange pouvant assurer une très bonne homogénéité et un dispositif de mise à pression atmosphérique et porté à 100° C, on introduit à travers une grille de un millimètre d'ouverture de maille, 36,750 kg d'acide citrique anhydre poudre et 63,250 kg de bicarbonate de sodium et on chauffe le mélange sous agitation énergique. Lorsque la température des produits atteint 40° C, on règle le thermostat de la double enveloppe à 75° C. Lorsque la température du mélange atteint 45 - 46° C, et que la température de la jaquette est à 75° C, on procède à l'addition de 160 ml d'eau sous forme pulvérisée tout en maintenant une vigoureuse agitation. On laisse réagir pendant 28 min 30 s. On diminue alors la vitesse d'agitation et on bloque la réaction par un séchage sous aspiration énergique ce qui se traduit par un abaissement de la pression résiduelle et de la température (de l'ordre de 5 à 10° C) celle-ci remontant lorsque la masse est sèche. A ce moment, on casse le vide et on calibre la masse par passage sur une grille de deux millimètres d'ouverture de maille.

On récupère ainsi 73,520 kg d'un couple avec excès de bicarbonate de sodium présentant une activité effervescente de 61,9% et correspondant à une composition de 26,3 % de citrate monosodique, 25,7% de citrate disodique et 48% de bicarbonate de sodium.

## EXEMPLE 17

Dans des conditions d'ambiance contrôlée en température (20° C) et en humidité relative (20%), on mélange intimement 1,1445 partie de chlorhydrate de cimétidine avec 15 parties du couple obtenu à la fin de l'exemple 16 et on répartit le mélange dans des sachets formés par scellage de feuilles de complexe Kraft 50 g, polyéthylène 12,5 g, aluminium 30 um et polyéthylène 25 g.

Pour évaluer la stabilité de la poudre ainsi conditionnée, les sachets ont été stockés pendant 1 semaine è 60° C et après cette période, aucun produit de dégradation n'a été décelé par chromatographie en phase liquide à haute performance.

Dans les mêmes conditions de conservation un mélange de
- 1,1445 partie de chlorhydrate de cimétidine
- 9,4875 parties de bicarbonate de sodium
- 5,5125 parties d'acide citrique anhydre poudre
a montré après 1 semaine à 60° C un taux de 40,9 % de N-carbamyl-N'-méthyl-N'-[2-(5-méthyl-4-imidazolylméthylthio)éthyl]-guanidine et 0,8% de N-méthyl-N'-[2-(5-méthyl-4-imidazolylméthylthio)éthyl]-guanidine.

Dans les mêmes conditions de conservation un mélange de
- 1,1445 parties de chlorhydrate de cimétidine
- 6,97 parties de citrate monosodique
- 8,03 parties de bicarbonate de sodium
a montré après une semaine à 60° C un taux de 5,4 % de N-carbamyl-N'-méthyl-N-[2-(5-méthyl-4-imidazolylméthylthio)éthyl]-guanidine.

Dans les mêmes conditions de conservation un mélange de
- 1,1445 partie de chlorhydrate de cimétidine
- 3,94 parties de citrate monosodique
- 3,86 parties de citrate disodique
- 7,20 parties de bicarbonate de sodium
présentant la même activité (61,9%) que le couple obtenu à la fin de l'exemple 16 a montré après 1 semaine à 60° C un taux de 4,9 % de N-carbamyl-N'-[2-(5-méthyl-4-imidazolylméthylthio)éthyl]-guanidine.

**Revendications pour les Etats Contractants BE, CH, FR, GB, IT, LI, LU, NL, SE:**

1. Procédé de préparation d'un couple effervescent dans lequel on mélange de l'acide citrique à une quantité au moins stoechiométrique de carbonate ou de bicarbonte alcalin et on granule le mélange homogénéisé, caractérisé en ce qu'on laisse réagir le mélange jusqu'à obtention d'un couple dans lequel l'acide citrique a été transformé en citrate mono- et di-alcalin dans un rapport compris entre environ 8/1 et environ 1/10 en poids, et on arrête à ce moment la réaction par séchage de la masse.

2. Procédé de préparation d'un couple effervescent suivant la revendication 1, caractérisé en ce que le rapport en poids est compris entre environ 2/1 et environ 6/10.

3. Procédé suivant la revendication 1 ou la revendication 2 dans lequel le mélange acide citrique/carbonate ou bicarbonate est stoechiométrique et la réaction est stoppée lorsque environ de 24 à 54% du potentiel anhydride carbonique est dégagé.

4. Procédé de préparation d'un couple effervescent suivant l'une quelconque des revendications 1 à 3, caractérisé en ce qu'on utilise du bicarbonate de sodium et que les citrates sont le citrate monosodique et le citrate disodique.

5. Composition pharmaceutique comprenant un couple effervescent préparé suivant l'une quelconque des revendications 1 à 4 et un antagoniste H2 de l'histamine avec un excipient non toxique pharmaceutiquement acceptable.

6. Composition pharmaceutique suivant la revendication 5 dans laquelle l'antagoniste H2 de l'histamine est la cimétidine, la Famotidine, la ranitidine ou la nizatidine.

7. Composition pharmaceutique suivant la revendication 5 dans laquelle l'antagoniste H2 de l'histamine est un dérivé de cyanoguanidine.

8. Composition pharmaceutique suivant la revendication 7 dans laquelle l'antagoniste H2 de l'histamine dérivé de cyanoguanidine est la cimétidine sous forme de base libre ou de chlorhydrate.

9. Composition pharmaceutique suivant l'une quelconque des revendications 5 à 8 dans laquelle la composition est sous forme de comprimé.

10. Composition pharmaceutique suivant l'une quelconque des revendications 5 à 9, caractérisée en ce qu'elle comprend également un antacide.

11. Composition pharmaceutique suivant la revendication 10, caractérisée en ce que l'antacide est un carbonate ou bicarbonate en excès de la quantité stoechiométrique.

12. Composition pharmaceutique suivant la revendication 11 dans laquelle l'antacide est du bicarbonate de sodium.

## Revendications pour les Etats Contractants AT, ES, GR:

1. Procédé de préparation d'un couple effervescent dans lequel on mélange de l'acide citrique à une quantité au moins stoechiométrique de carbonate ou de bicarbonate alcalin et on granule le mélange homogénéisé, caractérisé en ce qu'on laisse réagir le mélange jusqu'à obtention d'un couple dans lequel l'acide citrique a été transformé en citrate mono- et di-alcalin dans un rapport compris entre environ 8/1 et environ 1/10 en poids, et on arrête à ce moment la réaction par séchage de la masse.

2. Procédé de préparation d'un couple effervescent suivant la revendication 1, caractérisé en ce que le rapport en poids est compris entre environ 2/1 et environ 6/10.

3. Procédé suivant la revendication 1 ou la revendication 2 dans lequel le mélange acide citrique/carbonate ou bicarbonate est stoechiométrique et la réaction est stoppée lorsque environ de 23 à 54% du potentiel anhydride carbonique est dégagé.

4. Procédé de préparation d'un couple effervescent suivant l'une quelconque des revendications 1 à 3 caractérisé en ce qu'on utilise du bicarbonate de sodium et que les citrates sont le citrate monosodique et le citrate disodique.

5. Procédé de préparation d'une composition pharmaceutique combinant le produit d'une quelconque des revendications 1 à 4 avec un antagoniste H2 de l'histamine.

6. Procédé suivant la revendication 5 dans laquelle l'antagoniste H2 de l'histamine est la cimétidine, la famotidine, la ranitidine ou la nizatidine.

7. Procédé suivant la revendication 5 dans laquelle l'antagoniste H2 de l'histamine est un dérivé de cyanoguanidine.

8. Procédé suivant la revendication 7 dans laquelle l'antagoniste H2 de l'histamine dérivé de cyanoguanidine est la cimétidine sous forme de base libre ou de chlorhydrate.

## Claims for the Contracting States AT, ES, GR:

1. Process for the preparation of an effervescent system in which citric acid is mixed with an at least stoichiometric amount of alkali metal carbonate or bicarbonate and the homogenized mixture is granulated, characterized in that the mixture is allowed to react until a system is obtained in which the citric acid has been converted to mono-alkali metal citrate and di-alkali metal citrate in a ratio of between approximately 8/1 and approximately 1/10 by weight and at this time the reaction is stopped by drying the mass.

2. Process for preparing an effervescent system according to Claim 1, characterized in that the ratio by weight is between approximately 2/1 and approximately 6/10.

3. Process according to Claim 1 or Claim 2, in which the citric acid/carbonate or bicarbonate mixture is stoichiometric and the reaction is stopped when approximately from 23 to 54% of the potential carbon dioxide has been liberated.

4. Process for the preparation of an effervescent system according to any one of Claims 1 to 3, characterized in that sodium bicarbonate is used and in that the citrates are monosodium citrate and disodium citrate.

5. Process for the preparation of a pharmaceutical composition combining the product of any one of Claims 1 to 4 with a histamine H2 antagonist.

6. Process according to Claim 5, in which the histamine H2 antagonist is cimetidine, famotidine, ranitidine or nizatidine.

7. Process according to Claim 5, in which the histamine H2 antagonist is a cyanoguanidine derivative.

8. Process according to Claim 7, in which the histamine H2 antagonist derived from cyanoguanidine is cimetidine in the form of the free base or of the hydrochloride.

**Claims for the Contracting States BE, CH, FR, GB, IT, LI, LU, NL, SE:**

1. Process for the preparation of an effervescent system in which citric acid is mixed with an at least stoichiometric amount of alkali metal carbonate or bicarbonate and the homogenized mixture is granulated, characterized in that the mixture is allowed to react until a system is obtained in which the citric acid has been converted to mono-alkali metal citrate and di-alkali metal citrate in a ratio of between approximately 8/1 and approximately 1/10 by weight and at this time the reaction is stopped by drying the mass.

2. Process for preparing an effervescent system according to Claim 1, characterized in that the ratio by weight is between approximately 2/1 and approximately 6/10.

3. Process according to Claim 1 or Claim 2, in which the citric acid/carbonate or bicarbonate mixture is stoichiometric and the reaction is stopped when approximately from 24 to 54% of the potential carbon dioxide has been liberated.

4. Process for the preparation of an effervescent system according to any one of Claims 1 to 3, characterized in that sodium bicarbonate is used and in that the citrates are monosodium citrate and disodium citrate.

5. Pharmaceutical composition comprising an effervescent system prepared according to any one of Claims 1 to 4 and a histamine H2 antagonist with a pharmaceutically acceptable non-toxic excipient.

6. Pharmaceutical composition according to Claim 5 in which the histamine H2 antagonist is cimetidine, famotidine, ranitidine or nizatidine.

7. Pharmaceutical composition according to Claim 5, in which the histamine H2 antagonist is a cyanoguanidine derivative.

8. Pharmaceutical composition according to Claim 7, in which the histamine H2 antagonist derived from cyanoguanidine is cimetidine in the form of the free base or of the hydrochloride.

9. Pharmaceutical composition according to any one of Claims 5 to 8, in which the composition is in the form of a tablet.

10. Pharmaceutical composition according to any one of Claims 5 to 9, characterized in that it also contains an antacid.

11. Pharmaceutical composition according to Claim 10, characterized in that the antacid is a carbonate or bicarbonate in excess of the stoichiometric amount.

12. Pharmaceutical composition according to Claim 11, in which the antacid is sodium bicarbonate.

**Patentansprüche für die Vertragsstaaten BE, CH, FR, GB, IT, LI, LU, NL, SE:**

1. Verfahren zur Herstellung eines Brausepaares, bei dem man Citronensäure mit einer mindestens stöchiometrischen Menge Alkalicarbonat oder -bicarbonat vermischt und das homogenisierte Gemisch granuliert, dadurch gekennzeichnet, daß man das Gemisch reagieren läßt, bis ein Paar erhalten ist, in dem die Citronensäure in einem Gewichtsverhältnis zwischen etwa 8/1 und etwa 1/10 in Mono- und Dialkalicitrat umgewandelt ist, und zu diesem Zeitpunkt die Umsetzung durch Trocknen der Masse anhält.

2. Verfahren zur Herstellung eines Brausepaares nach Anspruch 1, dadurch gekennzeichnet, daß das Gewichtsverhältnis zwischen etwa 2/1 und etwa 6/10 liegt.

3. Verfahren nach Anspruch 1 oder Anspruch 2, in dem das Gemisch Citronensäure/Carbonat oder Bicarbonat stöchiometrisch ist und die Umsetzung angehalten wird, wenn etwa 24 bis 54% des möglichen Kohlensäureanhydrids entwickelt ist.

4. Verfahren zur Herstellung eines Brausepaares nach einem der Ansprüche 1 bis 3, dadurch gekennzeichnet, daß man Natriumbicarbonat verwendet und die Citrate Mononatriumcitrat und Dinatriumcitrat sind.

5. Arzneimittel, umfassend ein nach einem der Ansprüche 1 bis 4 hergestelltes Brausepaar und einen Histamin H2-Antagonisten mit einem nicht-toxischen, pharmazeutisch verträglichen Träger.

6. Arzneimittel nach Anspruch 5, in dem der Histamin H2-Antagonist Cimetidin, Famotidin, Ranitidin oder Nizatidin ist.

7. Arzneimittel nach Anspruch 5, in dem der Histamin H2-Antagonist ein Cyanoguanidin-Derivat ist.

8. Arzneimittel nach Anspruch 7, in dem der Histamin H2-Antagonist, der ein Cyanoguanidin-Derivat ist, Cimetidin in Form der freien Base oder des Chlorhydrats ist.

9. Arzneimittel nach einem der Ansprüche 5 bis 8, in dem das Mittel in verpreßter Form vorliegt.

10. Arzneimittel nach einem der Ansprüche 5 bis 9, dadurch gekennzeichnet, daß es auch ein Antacidum enthält.

11. Arzneimittel nach Anspruch 10, dadurch gekennzeichnet, daß das Antacidum ein Carbonat oder Bicarbonat im Überschuß zur stöchiometrischen Menge ist.

12. Arzneimittel nach Anspruch 11, in dem das Antacidum Natriumbicarbonat ist.

**Patentansprüche für die Vertragsstaaten AT, ES, GR:**

1. Verfahren zur Herstellung eines Brausepaares, bei dem man Citronensäure mit einer mindestens stöchiometrischen Menge Alkalicarbonat oder -bicarbonat vermischt und das homogenisierte Gemisch granuliert, dadurch gekennzeichnet, daß man das Gemisch reagieren läßt, bis ein Paar erhalten ist, in dem die Citronensäure in einem Gewichtsverhältnis zwischen etwa 8/1 und etwa 1/10 in Mono- und Dialkalicitrat umgewandelt ist, und zu diesem Zeitpunkt die Umsetzung durch Trocknen der Masse anhält.

2. Verfahren zur Herstellung eines Brausepaares nach Anspruch 1, dadurch gekennzeichnet, daß das Gewichtsverhältnis zwischen etwa 2/1 und etwa 6/10 liegt.

3. Verfahren nach Anspruch 1 oder Anspruch 2, in dem das Gemisch Citronensäure/Carbonat oder Bicarbonat stöchiometrisch ist und die Umsetzung angehalten wird, wenn etwa 23 bis 54% des möglichen Kohlensäureanhydrids entwickelt ist.

4. Verfahren zur Herstellung eines Brausepaares nach einem der Ansprüche 1 bis 3, dadurch gekennzeichnet, daß man Natriumbicarbonat verwendet und die Citrate Mononatriumcitrat und Dinatriumcitrat sind.

5. Verfahren zur Herstellung eines Arzneimittels, das das Produkt eines der Ansprüche 1 bis 4 mit einem Histamin $H_2$-Antagonisten verbindet.

6. Verfahren nach Anspruch 5, in dem der Histamin $H_2$-Antagonist Cimetidin, Famotidin, Ranitidin oder Nizatidin ist.

7. Verfahren nach Anspruch 5, in dem der Histamin $H_2$-Antagonist ein Cyanoguanidin-Derivat ist.

8. Verfahren nach Anspruch 7, in dem der von Cyanoguanidin abgeleitete Histamin $H_2$-Antagonist Cimetidin in Form der freien Base oder des Chlorhydrats ist.